# EUROPÄISCHE PATENTSCHRIFT

(11) **EP 1 124 792 B1**
(45) Veröffentlichungstag und Bekanntmachung des Hinweises auf die Patenterteilung: **04.08.2004**
(21) Anmeldenummer: 99971411.6
(22) Anmeldetag: 19.10.1999
(51) Int. Cl.: C07C 249/02

(54) **VERFAHREN ZUR HERSTELLUNG VON KETIMINEN**
METHOD OF PRODUCING KETIMINES
PROCEDE POUR LA PRODUCTION DE CETIMINES

(30) Priorität: 30.10.1998 CH 220198
(43) Veröffentlichungstag der Anmeldung: 22.08.2001
(73) Patentinhaber: Ciba Specialty Chemicals Holding Inc., 4057 Basel (CH)
(72) Erfinder: THOMMEN, Marc, CH-3125 Toffen (CH); HEROLD, Peter, CH-4057 Basel (CH)
(86) Internationale Anmeldenummer: PCT/EP1999/007894
(87) Internationale Veröffentlichungsnummer: WO 2000/026181

(56) Entgegenhaltungen:
- WO-A-99/36394
- DE-A- 2 348 577
- WELCH W M ET AL: "NONTRICYCLIC ANTIDEPRESSANT AGENTS DERIVED FROM CIS- AND TRANS-1-AMINO-4-ARYLTETRALINS" JOURNAL OF MEDICINAL CHEMISTRY,US,AMERICAN CHEMICAL SOCIETY. WASHINGTON, Bd. 27, Nr. 11, 1. November 1984 (1984-11-01), Seiten 1508-1515, XP000651829 ISSN: 0022-2623
- SARGES, R.: "Synthesis of Phenyl-Substituted 1-Aminotetralines" JOURNAL OR ORGANIC CHEMISTRY, Bd. 40, Nr. 9, 1975, Seiten 1216-1224, XP000872358

## Beschreibung

Die vorliegende Erfindung betrifft ein Verfahren zur Herstellung von Ketiminen, die sich als Ausgangsmaterialien zur Herstellung von pharmazeutischen Wirkstoffen wie beispielsweise Sertralin mit antidepressiven Eigenschaften eignen.

Die bis jetzt besten Verfahren zur Herstellung von Ketiminen sind beispielsweise in US-A-4,536,518 oder US-A-4,855,500 beschrieben.

Das in US-A-4,536,518 (Kolonnen 9/10, Beispiel 1(F)) offenbarte Verfahren zur Herstellung von Ketiminen ist dadurch gekennzeichnet, dass das Keton in einem aprotischen Lösungsmittel, wie beispielsweise Tetrahydrofuran mit Methylamin in Gegenwart von Titantetrachlorid unter Kühlung umgesetzt wird. Dieses Verfahren hat den Nachteil, dass mit dem leicht brennbaren Tetrahydrofuran und dem ökologisch nicht unbedenklichen Titantetrachlorid gearbeitet werden muss. Die Verfahrensführung ist zudem teuer, weil die Reaktion unter Kühlung erfolgt. Ein weiterer Nachteil dieses Verfahrens betrifft die Aufarbeitung. Das Produkt muss mit zusätzlichem Hexan ausgefällt werden.

Das in US-A-4,855,500 (Kolonnen 5/6, Anspruch 1) offenbarte Verfahren zur Herstellung von Ketiminen ist dadurch gekennzeichnet, dass das Keton in einem aprotischen Lösungsmittel, wie beispielsweise Methylenchlorid, Toluol oder Tetrahydrofuran mit wasserfreiem Methylamin in Gegenwart eines Molekularsiebs unter Kühlung umgesetzt wird.

Auch dieses Verfahren hat den Nachteil, dass wasserfrei mit ökologisch nicht unbedenklichen Lösungsmitteln wie beispielsweise Methylenchlorid oder mit leicht brennbaren Lösungsmitteln, wie beispielsweise Tetrahydrofuran, gearbeitet werden muss. Das eingesetzte Molekularsieb ist teuer und muss in einem zusätzlichen Schritt wieder recyclisiert werden. Ein weiterer Nachteil dieses Verfahrens ist, dass das Molekularsieb abgetrennt und das Produkt mit zusätzlichem Hexan ausgefällt werden muss.

Aus der WO-A-99/36394 ist ein Verfahren bekannt, worin die Umsetzung in Alkohol als Lösungsmittel, jedoch ohne die nachstehend genannten Katalysatoren, ausgeführt wird.

Es besteht deshalb weiterhin der Bedarf, ein effizientes Verfahren zur Herstellung von Ketiminen zu finden, das die oben aufgeführten Nachteile nicht aufweist und insbesondere in protischen Lösungsmitteln, wie beispielsweise Alkoholen funktioniert.

Die vorliegende Erfindung betrifft daher ein Verfahren zur Herstellung von Verbindungen der Formel 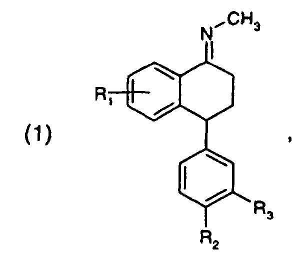 worin
- R₁, R₂ und R₃,: unabhängig voneinander Wasserstoff, Halogen, Trifluormethyl oder C₁-C₄-Alkoxy bedeuten, das dadurch gekennzeichnet ist, dass

(a) eine Verbindung der Formel 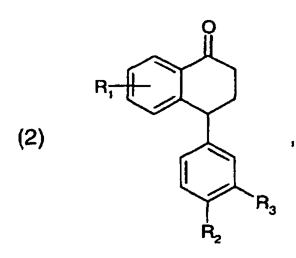 worin
   R₁, R₂ und R₃ die in Formel (1) genannten Bedeutungen haben, mit Methylamin in einem protischen Lösungsmittel umgesetzt wird, und
(b) der Reaktionsschritt (a) in Anwesenheit eines Katalysators durchgeführt wird, wobei der Katalysator eine Protonensäure, eine Lewis-Säure, ein Aluminiumsilikat, ein Ionenaustauscherharz, ein Zeolith, ein natürlich vorkommendes Schichtsilikat oder ein modifiziertes Schichtsilikat ist.

Halogen bedeutet beispielsweise Chlor, Brom oder lod. Bevorzugt ist Chlor.

Alkoxy mit bis zu 4 Kohlenstoffatomen bedeutet einen verzweigten oder unverzweigten Kohlenwasserstoffrest wie beispielsweise Methoxy, Ethoxy, Propoxy, Isopropoxy, n-Butoxy, Isobutoxy oder tert-Butoxy. Bevorzugt ist Methoxy.

Erfindungsgemäss bevorzugt ist ein Verfahren, worin die erhaltene Verbindung der Formel (1) einer Reinigung durch Umkristallisation unterzogen wird.

Von Interesse ist ein Verfahren zur Herstellung von Verbindungen der Formel (1 ), worin
- R₁: Wasserstoff oder Chlor bedeutet.

Ebenfalls von Interesse ist ein Verfahren zur Herstellung von Verbindungen der Formel (1), worin
- R₂ und R₃,: unabhängig voneinander Wasserstoff, Chlor oder Brom bedeuten.

Von besonderem Interesse ist ein Verfahren zur Herstellung von Verbindungen der Formel (1), worin
- R₁: Wasserstoff und
- R₂ und R₃: Chlor bedeuten.

Speziell von besonderem Interesse ist ein Verfahren zur Herstellung von Verbindungen der Formel (1), worin das protische Lösungsmittel einen a-wertigen Alkohol bedeutet, wobei a die Zahl 1, 2, 3 oder 4 bedeutet.

Bevorzugt ist ein Verfahren zur Herstellung von Verbindungen der Formel (1), worin das protische Lösungsmittel eine Verbindung der Formel

(3) X(OH)ₐ

bedeutet, worin
- a: 1, 2, 3 oder 4 bedeutet, und
wenn a 1 ist,
- X: C₁-C₈-Alkyl, C₅-C₈-Cycloalkyl oder -CH₂CH₂(OCH₂CH₂)_{b}R₄,
- b: 0, 1 oder 2, und
- R₄: C₁-C₄-Alkoxy bedeuten, oder
wenn a 2 ist,
- X: C₂-C₈-Alkylen oder -CH₂CH₂(OCH₂CH₂)_{b}- bedeutet, wobei b die obige Bedeutung hat,
oder
wenn a 3 ist,
- X: C₃-C₈-Alkantriyl oder N(CH₂CH₂-)₃ bedeutet, oder
wenn a 4 ist,
- X: C₄-C₈-Alkantetrayl bedeutet.

Alkyl mit bis zu 8 Kohlenstoffatomen bedeutet einen verzweigten oder unverzweigten Kohlenwasserstoffrest wie beispielsweise Methyl, Ethyl, n-Propyl, Isopropyl, n-Butyl, Isobutyl, tert-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl oder Isooctyl.

C₅-C₈-Cycloalkyl bedeutet beispielsweise Cyclopentyl, Cycloheptyl oder vorzugsweise Cyclohexyl.

Alkoxy mit bis zu 4 Kohlenstoffatomen bedeutet einen verzweigten oder unverzweigten Kohlenwasserstoffrest wie beispielsweise Methoxy, Ethoxy, Propoxy, Isopropoxy, n-Butoxy, Isobutoxy oder tert-Butoxy. Bevorzugt ist Methoxy.

C₂-C₈-Alkylen bedeutet einen verzweigten oder unverzweigten Rest wie beispielsweise Ethylen, Propylen, Trimethylen, Tetramethylen, Pentamethylen, Hexamethylen, Heptamethylen oder Octamethylen.

Alkantriyl mit 3 bis 8 Kohlenstoffatomen leitet sich von einem Alkan mit 3 bis 8 Kohlenstoffatomen ab, worin 3 Wasserstoffatome fehlen und bedeutet beispielsweise 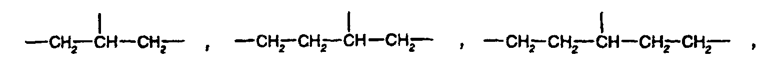 oder 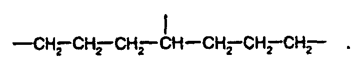 Bevorzugt ist Glyceryl.

Alkantetrayl mit 4 bis 8 Kohlenstoffatomen leitet sich von einem Alkan mit 4 bis 8 Kohlenstoffatomen ab, worin 3 Wasserstoffatome fehlen, und bedeutet beispielsweise 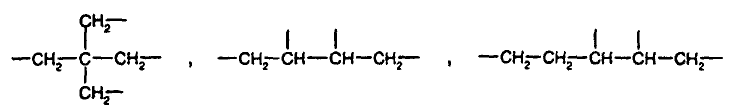 oder 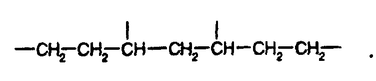 Bevorzugt ist Pentaerythrityl.

Eine bevorzugte Bedeutung von X (für a = 1) ist beispielsweise C₁-C₆-Alkyl, insbesondere C₁-C₄-Alkyl, z.B. Ethyl oder Isopropyl.

Eine bevorzugte Bedeutung von X (für a = 2) ist beispielsweise C₂-C₆-Alkylen, insbesondere C₂-C₄-Alkylen, z.B. Ethylen.

Von besonderem Interesse ist ein Verfahren zur Herstellung von Verbindungen der Formel (1), worin das protische Lösungsmittel eine Verbindung der Formel

(3) X(OH)ₐ

bedeutet, worin
- a: 1 oder 2 bedeutet, und
wenn a 1 ist,
- X: C₁-C₄-Alkyl oder C₅-C₆-Cycloalkyl bedeutet, oder
wenn a 2 ist,
- X: C₂-C₄-Alkylen bedeutet.

Besonders bevorzugt ist ein Verfahren zur Herstellung von Verbindungen der Formel (1), worin das protische Lösungsmittel Methanol, Ethanol, Isoproponanol, n-Butanol, Ethylenglykol, Methylcellosolve, Cyclohexanol, Diethylenglykol oder Triethanolamin bedeuet.

Speziell bevorzugt ist ein Verfahren zur Herstellung von Verbindungen der Formel (1), worin das protische Lösungsmittel Ethanol oder Isopropanol bedeutet.

Die Ausgangsverbindungen der Formel (2) sind bekannt oder können in Analogie zu den in der US-4,536,518 beschriebenen Verfahren hergestellt werden.

Bevorzugte Reaktionsbedingungen des erfindungsgemässen Verfahrens (Reaktionsschritt (a)) sind die folgenden:

Die Reaktion kann bei Raumtemperatur oder erhöhter Temperatur, insbesondere Temperaturen von 20 bis 100°C, z.B. 25 bis 65°C, gegebenenfalls unter leichtem Druck durchgeführt werden.

Besonders bevorzugt wird die Reaktion mit einem grossen molaren Überschuss an Methylamin durchgeführt. Besonders bevorzugt ist deshalb ein Verfahren zur Herstellung von Verbindungen der Formel (1), worin das molare Mengenverhältnis der Verbindung der Formel (2) zum Methylamin 1:1 bis 1:100, insbesondere 1:1,05 bis 1:50, z.B. 1:1,5 bis 1:15, beträgt.

Das Methylamin kann in Form von Methylamin-Gas oder als Lösung in einem Alkohol wie beispielsweise Ethanol eingesetzt werden.

Bevorzugte Katalysatoren für das Verfahren zur Herstellung der Verbindungen der Formel (1) sind Protonensäuren, Lewis-Säuren, Aluminiumsilikate, Ionenaustauscherharze, Zeolithe, natürlich vorkommendes Schichtsilikate oder modifizierte Schichtsilikate.

Geeignete Protonensäuren sind beispielsweise Säuren von anorganischen oder organischen Salzen, wie z.B. Salzsäure; Schwefelsäure; Phosphorsäure oder Sulfonsäuren, wie beispielsweise Methansulfonsäure, p-Toluolsuifonsäure oder Campher-10-sulfonsäure.

Eine geeignete Lewis-Säure ist beispielsweise Scandiumtristriflat [Sc(OTf)₃].

Geeignete Aluminiumsilikate sind beispielsweise solche, die in der Petrochemie weit verbreitet sind und auch als amorphe Aluminiumsilikate bezeichnet werden. Diese Verbindungen enthalten ca. 10-30 % Siliciumoxid und 70-90 % Aluminiumoxid.

Geeignete Ionenaustauscherharze sind beispielsweise Styrol-Divinylbenzol-Harze, die noch Sulfosäuregruppen tragen, wie z.B. Amberlie 200® und Amberlyst® von Rohm und Haas oder Dowex 50® von Dow Chemicals; perfluorierte Ionenaustauscherharze, wie z.B. NafionH® von DuPont; oder andere supersaure Ionenaustauscherharze wie von T. Yamaguchi, Applied Catalysis, 61, 1-25 (1990) oder M. Hino et al., J. Chem. Soc. Chem. Commun. 1980, 851-852 beschrieben.

Geeignete Zeolithe sind beispielsweise solche, die in der Petrochemie als Cracking-Katalysatoren weit verbreitet sind und als kristalline Silicium-Aluminiumoxide mit unterschiedlicher Kristallstruktur bekannt sind. Besonders bevorzugt sind die Faujasite der Firma Union Carbide wie beispielsweise Zeolith X® Zeolith Y® und ultrastabiler Zeolith Y®; Zeolith Beta® und Zeolith ZSM-12® von der Firma Mobil Oil Co.; und Zeolith Mordenit® von der Firma Norton.

Geeignete natürlich vorkommende Schichtsilikate werden auch als "Saure Erden" bezeichnet und sind beispielsweise Bentonite oder Montmorillonite, die grosstechnisch abgebaut, gemahlen, mit Mineralsäuren behandelt und kalziniert werden. Besonders geeignete natürlich vorkommende Schichtsilikate sind die Fulcat®-Typen der Firma Laporte Adsorbents Co., wie z.B. Fulcat 22A®, Fulcat 22B®, Fulcat 20®, Fulcat 30® oder Fulcat 40®; oder die Fulmont®-Typen der Firma Laporte Adsobents Co., wie z.B. Fulmont XMP-3® oder Fulmont XMP-4®. Ein speziell bevorzugter Katalysator für das erfindungsgemässe Verfahren ist Futcat 22B®. Die anderen Fulcat®-Typen und Fulmont®-Typen sind aber ebenfalls in diese bevorzugte Klasse einzuordnen, weil es nur geringfügige Unterschiede zwischen den einzelnen Typen gibt, wie beispielsweise in der Anzahl der sauren Zentren.

Modifizierte Schichtsilikate werden auch als "Pillared Clays" bezeichnet und leiten sich von den oben beschriebenen natürlich vorkommenden Schichtsilikaten ab, indem sie zwischen den Silicatschichten noch Oxide von beispielsweise Zirkon, Eisen, Zink, Nickel, Chrom, Coalt oder Magnesium enthalten. Dieser Katalysator-Typ ist in der Literatur, wie z.B. von J. Clark et al., J. Chem. Soc. Chem. Commun. 1989, 1353-1354 beschrieben, weit verbreitet, wird aber nur von sehr wenigen Firmen hergestellt. Besonders bevorzugte modifizierte Schichtsilikate sind beispielsweise Envirocat EPZ-10®, Envirocat EPZG® oder Envirocat EPIC® von der Firma Contract Chemicals.

Speziell bevorzugt ist auch ein Verfahren zur Herstellung von Verbindungen der Formel, worin der Katalysator eine Sulfonsäure, insbesondere p-Toluolsulfonsäure, Methansulfonsäure oder Campher-1 0-sulfonsäure bedeutet.

Das molare Mengenverhältnis des eingesetzten Katalysators zum eingesetzten Methylamin beträgt zweckmässig 0,001:1 bis 1:1, insbesondere 0,01:1 bis 0,5:1, z.B. 0,05:1 bis 0,1:1.

Das 1:1-molare Mengenverhältnis des Katalysators zum Methylamin bedeutet auch, dass das Methylamin auch in Form eines Salzes, wie beispielsweise des Methylaminhydrochbrids in dem erfindungsgemässen Verfahren eingesetzt werden kann.

Von besonderem Interesse ist auch ein Verfahren zur Herstellung von Verbindungen der Formel (1), worin die Verbindung der Formel (1) während der Herstellung kontinuierlich aus dem Reaktionsmedium auskristallisiert und anschliessend abfiltriert wird.

Von speziellem Interesse ist auch ein Verfahren zur Herstellung von Verbindungen der Formel (1 ), worin das Filtrat für eine weitere Umsetzung zur Herstellung von Verbindungen der Formel (1) eingesetzt wird. Dabei werden die verbrauchten Mengen an der Verbindung der Formel (2) und Methylamin ergänzt. Bevorzugt sind 2 bis 10 Recyclierungen des Filtrats.

Das vorliegende erfindungsgemässe Verfahren eignet sich demzufolge auch als kontinuierliches Verfahren zur Herstellung der Verbindungen der Formel (1).

Das während des Verfahrensgebildete Wasser kann gegebenenfalls mit einem zusätzlichen Wasserbinder wie beispielsweise Molekularsieben oder Orthoester, z.B. ortho-Ameisensäuretrimethylester, gebunden werden.

Der Reinigungsschritt (b) wird in einem protischen, insbesondere alkoholischen Lösungsmittel durchgeführt. Besonders bevorzugte Alkohole entsprechen der Formel (3), insbesondere Ethanol oder Isopropanol.

In einer besonders bevorzugten Ausführungsform wird der Reinigungsschritt (b) im gleichen Lösungsmittel wie der Reaktionsschritt (a) durchgeführt.

In einer bevorzugten Verfahrensvariante erfolgt die Reinigung durch Umkristallisieren von Sertralin-Imin (Verbindung der Formel (1)) unter Rückfluss. Dazu wird in einem geeigneten Reaktionsgefäss mit Rührer und Rückflusskühler das isomerenreine Sertralin-Imin, das gewöhnlich mit 2 bis 10 % Sertralon und 0,01 bis 0,3 % Sulfonsäure kontaminiert ist, in einem geeigneten Alkohol vorgelegt. Die Reaktionsmasse wird in einer Inertgasatmosphäre unter Rühren auf Rückflusstemperatur erwärmt, bis eine klare Lösung vorliegt. Die Lösung wird auf die entsprechende Isolationstemperatur abgekühlt, wobei das Produkt langsam ausfällt. Die Suspension wird filtriert, der Filterkuchen mit dem Lösungsmittel gewaschen und getrocknet. Die Iminausbeute beträgt zwischen 80-90 %, mit einem Sertralon-Anteil von 0,1 bis 0,3% (HPLC), einer Katalysatorkontamination von ≤ 0,001 und 0,1 bis 0,3 % Wasseranteil.

In einer weiteren Verfahrensvariante erfolgt die Umkristallisation von Sertralin-Imin unter Druck. Dazu wird in einem geeigneten Druckreaktor mit Rührer das rohe Sertralin-Imin und das Lösungsmittel vorgelegt. Der Reaktor wird mit Stickstoff inertisiert und verschlossen. Der Rührer wird gestartet und das Reaktionsgemisch auf die gewünschte Reaktionstemperatur erwärmt, bis eine klare Lösung vorliegt. Die Lösung wird auf die entsprechende Isolations-temperatur abgekühlt, wobei das Produkt langsam ausfällt. Die Suspension wird filtriert, der Filterkuchen mit dem Lösungsmittel gewaschen und getrocknet.

Die Lösungstemperaturen in den gewählten Alkoholen liegen im Bereich von 50 bis 150°C, vorzugsweise im Bereich von 70 bis 140°C.

Entsprechend der Siedepunkte der angegebenen Lösungsmittel können die Löseexperimente unter Normaldruck oder erhöhtem Druck, normalerweise aber unter Rückfluss, durchgeführt werden.

Für Lösetemperaturen oberhalb des Siedepunktes können die Löseexperimente unter Druck durchgeführt werden, normalerweise im Bereich von 0 bis 10 bar Überdruck, bevorzugt im Bereich 0 bis 3 bar Überdruck.

Die Abkühlgradienten liegen im Bereich von 0,05 bis 10, vorzugsweise 0,1 bis 1°C/min.

Die Isofationstemperaturen liegen im Bereich von -20 bis 40, vorzugsweise 0 bis 25°C.

Die Konzentrationen von rohem Sertralin-Imin in der klaren Lösung liegen im Bereich von 5 bis 40, vorzugsweise 15 bis 20 Gew.-%.

Während des Prozesses können Adsorbentien wie Aktivkohle oder Adsorberharze zur Entfernung färbender Verunreinigungen zugesetzt werden. Diese werden in Mengen von 1 bis 10 % der klaren Lösung zugegeben und vor dem Kristallisationsprozess heiss durch Filtration entfernt.

Die vorliegende Erfindung betrifft auch ein Verfahren zur Herstellung von optisch reinem (cis)- und/oder (trans)-Sertralin oder enantiomerenangereicherten Mischungen von (cis)- und (trans)- Sertralin. Das Verfahren ist durch die folgenden Reaktionsschritte (I)-(III) gekennzeichnet:
(I) Umsetzung von reinem Sertralin-Keton der Formel (2) zum Sertralin-Imin der Formel (1) gemäss dem Verfahren nach Anspruch 1,
(II) nachfolgende cis-selektive Hydrierung mit Edelmetallkatalysatoren oder weiteren Katalysatoren auf Kupfer- oder Nickelbasis zu cis-Sertralin angereicherten Mischungen von racemischem cis- und trans-Sertralin,
(III) nachfolgende Racematspaltung auf Mandelsäurebasis zur selektiven Herstellung des gewünschten enantiomerenreinen cis-Isomeren.

Ausgehend von reinem Sertralin-Keton wird Sertralin-Imin gemäss dem in Anspruch 1 beschriebenen Verfahren hergestellt. Das Imin wird in einer nachfolgenden cis-selektiven Hydrierung mit Edelmetallkatalysatoren oder weiteren Katalysatoren auf Kupfer- oder Nickelbasis der verschiedensten Träger, wie z.B. Kohle, Alox, Silica, Calciumcarbonat, Bariumcarbonat, Bariumsulfat etc. zu cis-Sertralin angereicherten Mischungen von racemischem cis- und trans-Sertralin überführt.

In einer nachfolgenden Racematspaltung auf Mandelsäurebasis kann selektiv das gewünschte enantiomerenreine cis-Isomere kristallisiert werden.

Das optisch reine Amin wird mit Natronlauge freigesetzt und als Hydrochlorid in geeigneten Lösungsmitteln in die gewünschte polymorphe Form überführt.

Die folgenden Beispiele erläutern die Erfindung weiter. Angaben in Teilen oder Prozenten beziehen sich auf das Gewicht.

### Beispiel 1: Herstellung der Verbindung der Formel (101) mit p-Toluolsulfonsäure als Katalysator

In einen 50 ml Rundkolben werden 20 ml Ethanol vorgelegt. Dazu werden nacheinander unter Rühren 0.60 g 3.44 mMol) getrocknete p-Toluolsulfonsäure (getrocknet bei 100-110°C und 100-200 mbar), 3,16 g (34,4 mMol) einer 33 % ethanolischen Methylamin-Lösung und 5,0 g (17,2 mMol) 4-(3,4-Dichlorphenyl)-3,4-dihydro-1(2H)-naphthalinon (hergestellt gemäss US-A-4,536,518, Beispiel 1(E)) gegeben. Der Rundkolben wird anschliessend mit einem Stopfen dicht verschlossen und in einem vorgeheizten Ölbad auf 60°C erhitzt. Nach ca. 30 Minuten wird während 1 bis 4 Minuten eine klare Lösung beobachtet. Anschliessend beginnt die Kristallisation des Produkts der Formel (101). Nach ca. 3 Stunden sind über 95 % Umsatz erreicht (HPLC). Das Reaktionsgemisch wird abgekühlt und filtriert. Der Rückstand wird dreimal mit je 25 ml Ethanol gewaschen und anschliessend im Vakuumtrockenschrank bei ca. 70°C/0,1-0,2 mbar getrocknet. Es resultieren 14,57 g (87 %) der Verbindung der Formel (101).

Smp.: 145-147°C.

### Beispiel 2: Herstellung der Verbindung der Formel (101) unter Wiederverwendung des Filtrats (Recyclierungverfahren) mit p-Toluolsulfonsäure als Katalysator

In einen 50 ml Rundkolben werden 20 ml Ethanol vorgelegt. Dazu werden nacheinander unter Rühren 0,6g (3,44 mMol) getrocknete p-Toluolsulfonsäure (getrocknet bei 100-110°C und 100-200 mbar), 12,9 g (140,2 mMol) einer 33 % ethanolischen Methylamin-Lösung und 5,0 g (17,2 mMol) 4-(3,4-Dichlorphenyl)-3,4-dihydro-1(2H)-naphthalinon (hergestellt gemäss US-A-4,536,518, Beispiel 1 (E)) gegeben. Der Rundkolben wird anschliessend mit einem Stopfen dicht verschlossen und in einem vorgeheizten Ölbad auf 30°C erhitzt. Bei dieser Temperatur wird nie eine klare Lösung beobachtet. Nach ca. 5 Stunden und 40 Minuten sind über 95 % Umsatz erreicht (HPLC). Das Reaktionsgemisch wird abgekühlt und filtriert. Der Rückstand wird einmal mit 33 % ethanolischer Methylamin-Lösung gewaschen. Das Filtrat (Reaktionsmedium) wird erneut mit 5,0 g (17,2 mMol) 4-(3,4-Dichlorphenyl)-3,4-dihydro-1(2H)-naphthalinon [hergestellt gemäss U.S. 4,536,518, Beispiel 1(E)] versetzt und wie oben beschrieben einem weiteren Reaktionszyklus unterworfen. Nach drei weiteren Reaktionszyklen werden die Rückstände vereinigt, dreimal mit Ethanol gewaschen und anschliessend im Vakuumtrockenschrank bei ca. 70°C/0,1-0,2 mbar getrocknet.

Es resultieren 22,5 g (86 %) der Verbindung der Formel (101), Smp. 145-147°C.

### Beispiel 3: Herstellung der Verbindung der Formel (101) mit Scandiumtristriflat als Katalysator und Montmorillonit als Wasserbinder

In einen 50 ml Rundkolben werden 60 ml Ethanol vorgelegt. Dazu werden nacheinander unter Rühren 14,2 g (155 mMol) einer 33 % ethanolischen Methylamin-Lösung, 3,0 g getrocknetes G62 Montmorillonit (Wasserbinder), 50 mg (0,3 %, 0,13 mMol) Sc(OTf)₃ (Scandiumtristriflat) und 15,0 g (55 mMol) 4-(3,4-Dichlorphenyl)-3,4-dihydro-1(2H)-naphthalinon (hergestellt gemäss US-A-4,536,518, Beispiel 1(E)) gegeben. Der Rundkolben wird anschliessend mit einem Stopfen dicht verschlossen und in einem vorgeheizten Ölbad auf 60°C erhitzt. Nach ca. 20 Stunden sind über 95 % Umsatz erreicht (HPLC). Das Reaktionsgemisch wird abgekühlt und filtriert. Der Rückstand wird in Tetrahydrofuran aufgenommen. Das unlösliche Montmorillonit wird abfiltriert und mit Tetrahydrofuran gewaschen. Das Filtrat wird am Vakuumrotationsverdampfer eingeengt.

Der Rückstand liefert 13,5 g (86 %) der Verbindung der Formel (101).

Smp.: 145-147°C.

### Beispiel 4: Herstellung der Verbindung der Formel (101) mit Scandiumtristriflat als Katalysator und mit ortho-Ameisensäuretrimethylester als Wasserbinder

In einen 50 ml Rundkolben werden 20 ml Ethanol vorgelegt. Dazu werden nacheinander unter Rühren 4,73 g (50 mMol) einer 33 % ethanolischen Methylamin-Lösung, 2,55g -ortho-Ameisensäuretrimethylester (Wasserbinder), 100 mg (2,0 %, 0,26 mMol) Sc(OTf)₃ [Scandiumtristriflat] und 5,0 g (17,2 mMol) 4-(3,4-Dichlorphenyl)-3,4-dihydro-1(2H)-naphthalinon (hergestellt gemäss US-A-4,536,518, Beispiel 1(E)) gegeben. Der Rundkolben wird anschliessend mit einem Stopfen dicht verschlossen und in einem vorgeheizten Ölbad auf 60°C erhitzt. Nach ca. 6 Stunden sind über 95 % Umsatz erreicht (HPLC). Das Reaktionsgemisch wird abgekühlt und filtriert. Der Rückstand wird dreimal mit je 4 ml Ethanol gewaschen und anschliessend im Vakuumtrockenschrank bei ca. 70°C/0,1-0,2 mbar getrocknet. Es resultieren 4,5 g (86 %) der Verbindung der Formel(101).

Smp.: 145-147°C.

### Beispiele 5 bis 11: Herstellung von Sertralin-Imin mit verschiedenen Lösungsmittel-/Katalysatorkombinationen

In einem 50 ml Rundkolben werden jeweils 20 g des Lösungsmittels (a) vorgelegt. Zu 2,9 g einer 33%igen ethanolischen Methylamin-Lösung werden 0,33 Äquivalente (relativ zum eingesetzten Keton) des Katalysators (b) hinzugegeben. Man erhitzt 4 h bei der jeweils angegebenen Temperatur. Man entnimmt eine HPLC-Probe, erhitzt für weitere 16 Stunden und filtriert das feste Produkt der Formel (101). Abschliessend werden HPLC-Proben sowohl vom Produkt als auch der Mutterlauge entnommen.

Die Versuchsparameter und Ergebnisse sind in Tabelle 1 aufgeführt:

**Tabelle 1:**

| Beispiel | Temp. [°C] | Lösungsmittel (a) | Katalysator (b) | Ausbeute nach 4 h [%] HPLC | Isolierte Ausbeute [%]/ kontaminiert mit % Keton | rel. Imin-Konzentration in der Mutterlauge [%], HPLC |
|---|---|---|---|---|---|---|
| 5 | 60 | Isopropanol | p-TsOH | 93 | 81/4,5 | <1 |
| 6 | 30 | Ethanol | Methansulfonsäure | 46 | 84/3,2 | 6 |
| 7 | 30 | Isopropanol | Methansulfonsäure | 29 | 86/8,5 | 6 |
| 8 | 60 | Triethanolamin | p-TsOH | 92 | 77/4,0 | 28 |
| 9 | 60 | Ethylenglycol | p-TsOH | 93 | 84/3,8 | <20 |
| 10 | 60 | Cyclohexanol | p-TsOH | 81 | 64/12 | 18 |
| 11 | 30 | Ethanol | Camphersulfonsäure | 49 | 80/2,9 | 17 |

### Beispiel 12: Herstellung von Sertralin-Imin mit Methansulfonsäure als Katalysator

Sertralon (240,0 g; 0,825 mol) wird mit Ethanol_{abs} (800 ml) in einem geeigneten Reaktionsbehälter, ausgerüstet mit Rührer und Gas-Überleitung, vorgelegt.

Die Suspension wird auf 0°C gekühlt und Methylamin (55,0 g; 1,762 mol) wird unter Niveau, d.h. unterhalb der Oberfläche des Lösungsmittels, eingeleitet. Methansulfonsäure (10 ml) wird dann mit einer Spritze während 5 min zudosiert.

Die Reaktionsmasse wird aufgeheizt, 3 Stunden bei 50°C und 1 Stunde bei 70°C gerührt, um eine Konversion zum Imin (> 94 %) zu bekommen.

Die Reaktionsmischung wird dann auf 10°C gekühlt, filtriert und mit kaltem Ethanol gewaschen (2 x 250 ml). Der rohe Filterkuchen wird über Nacht unter Vakuum getrocknet und liefert 213 g trockenes N-Methyl-sertralin-Imin.

Qualität und Ausbeute des erhaltenen Imins (Bestimmung mittels HPLC):
88% Ausbeute an Sertralin.

Das Imin enthält:
1,8 % Sertralon
0,1 % Wasser
0,05 % Methansulfonsäure (bestimmt mit CE)

### Beispiel 13: Reinigung von Sertralin-Imin durch Umkristallisation aus Ethanol:

In einem geeigneten Reaktionsgefäss mit Rührer, Stickstoffüberleitung und Rückflusskühler werden 12 g Sertralin-Imin aus Beispiel 12 in 200 ml Ethanol vorgelegt. Der Rührer wird gestartet und das Reaktionsgemisch auf Rückflusstemperatur erwärmt, bis eine klare Lösung vorliegt. Die Lösung wird langsam auf 20°C abgekühlt, wobei das Produkt ausfällt. Die Suspension wird filtriert, der Filterkuchen mit dem Lösungsmittel gewaschen und getrocknet.

Man erhält 10,6 g Sertralin-Imin mit folgender Zusammensetzung (HPLC):
88% Ausbeute an Sertralin-Imin

Das Imin enthält:
0,2 % Sertralon
< 0,05 % Wasser
< 0,001% Methansulfonsäure (bestimmt mit CE).

Durch die Umkristallisation können sowohl die Produktreinheit verbessert als auch störende Verunreinigungen wie Wasser oder Katalysator-Reste abgetrennt werden.

## Patentansprüche

1. Verfahren zur Herstellung von Verbindungen der Formel 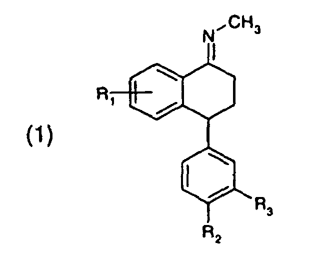 worin
R₁, R₂ und R₃ unabhängig voneinander Wasserstoff, Halogen, Trifluormethyl oder C₁-C₄-Alkoxy bedeuten,
**dadurch gekennzeichnet, dass**
(a) eine Verbindung der Formel 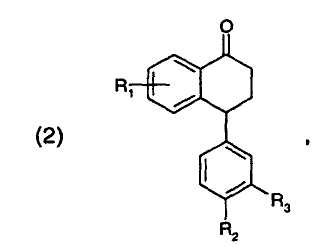 worin
R₁, R₂ und R₃ die in Formel (1) genannten Bedeutungen haben, mit Methylamin in einem protischen Lösungsmittel umgesetzt wird, und
(b) der Reaktionsschritt (a) in Anwesenheit eines Katalysators durchgeführt wird, wobei der Katalysator eine Protonensäure, eine Lewis-Säure, ein Aluminiumsilikat, ein Ionenaustauscherharz, ein Zeolith, ein natürlich vorkommendes Schichtsilikat oder ein modifiziertes Schichtsilikat ist.

2. Verfahren gemäss Anspruch 1, **dadurch gekennzeichnet, dass**
R₁ Wasserstoff oder Chlor bedeutet.

3. Verfahren gemäss einem der Ansprüche 1 und 2, **dadurch gekennzeichnet, dass**
R₂ und R₃ unabhängig voneinander Wasserstoff, Chlor oder Brom bedeuten.

4. Verfahren gemäss einem der Ansprüche 1 bis 3, **dadurch gekennzeichnet, dass**
R₁ Wasserstoff und
R₂ und R₃ Chlor
bedeuten.

5. Verfahren gemäss einem der Ansprüche 1 bis 4, **dadurch gekennzeichnet, dass** das protische Lösungsmittel einen a-wertigen Alkohol bedeutet, wobei a die Zahl 1, 2, 3 oder 4 bedeutet.

6. Verfahren gemäss Anspruch 5, **dadurch gekennzeichnet, dass** das protische Lösungsmittel eine Verbindung der Formel
(3) X(OH)ₐ
bedeutet, worin
a 1, 2, 3 oder 4 bedeutet, und
wenn a 1 ist,
X C₁-C₈-Alkyl, C₅-C₈-Cycloalkyl oder -CH₂CH₂(OCH₂CH₂)_{b}R₄ bedeutet,
b 0, 1 oder 2, und
R₄ C₁-C₄-Alkoxy bedeuten, oder
wenn a 2 ist,
X C₂-C₈-Alkylen oder -CH₂CH₂(OCH₂CH₂)_{b}- bedeutet, wobei b die obige Bedeutung hat, oder
wenn a 3 ist,
X C₃-C₈-Alkantriyl oder N(CH₂CH₂-)₃ bedeutet, oder
wenn a 4 ist,
X C₄-C₈-Alkantetrayl bedeutet.

7. Verfahren gemäss Anspruch 6, **dadurch gekennzeichnet, dass** das protische Lösungsmittel Methanol, Ethanol, Isoproponanol, n-Butanol, Ethylenglykol, Methylcellosolve, Cyclohexanol, Diethylenglykol oder Triethanolamin ist.

8. Verfahren gemäss Anspruch 7, **dadurch gekennzeichnet, dass** das protische Lösungsmittel Ethanol oder Isopropanol ist.

9. Verfahren gemäss einem der Ansprüche 1 bis 8, **dadurch gekennzeichnet, dass** der Katalysator eine Sulfonsäure bedeutet.

10. Verfahren gemäss Anspruch 9, **dadurch gekennzeichnet, dass** der Katalysator p-Toluolsulfonsäure, Methansulfonsäure oder Campher-10-sulfonsäure ist.

11. Verfahren gemäss Anspruch 1, **dadurch gekennzeichnet, dass** das Filtrat für eine weitere Umsetzung zur Herstellung der Verbindung der Formel (1) eingesetzt wird.

12. Verfahren gemäss einem der Ansprüche 1 bis 11, **dadurch gekennzeichnet, dass** das molare Mengenverhältnis der Verbindung der Formel (2) zum Methylamin 1:1 bis 1:100 beträgt.

## Claims

1. A process for the preparation of a compound of formula 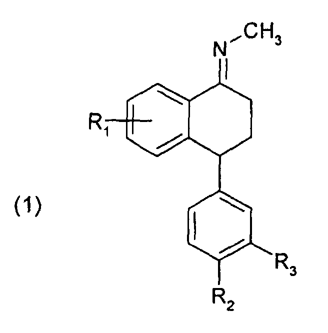 wherein
R₁, R₂ and R₃ are each independently of the others hydrogen, halogen, trifluoromethyl or C₁-C₄alkoxy, in which process
(a) a compound of formula 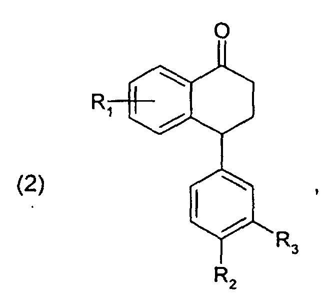 wherein
R₁, R₂ and R₃ are as defined for formula (1), is reacted with methylamine in a protic solvent, and
(b) reaction step (a) is carried out in the presence of a catalyst, the catalyst being a protonic acid, a Lewis acid, an aluminium silicate, an ion exchange resin, a zeolite, a naturally occurring layer silicate or a modified layer silicate.

2. A process according to claim 1, wherein R₁ is hydrogen or chlorine.

3. A process according to either claim 1 or claim 2, wherein R₂ and R₃ are each independently of the other hydrogen, chlorine or bromine.

4. A process according to any one of claims 1 to 3, wherein R₁ is hydrogen and R₂ and R₃ are chlorine.

5. A process according to any one of claims 1 to 4, wherein the protic solvent is an a-hydric alcohol wherein a is the number 1, 2, 3 or 4.

6. A process according to claim 5, wherein the protic solvent is a compound of formula
(3) X(OH)ₐ
wherein
a is 1, 2, 3 or 4, and
when a is 1,
X is C₁-C₈alkyl, C₅-C₈cycloalkyl or -CH₂CH₂(OCH₂CH₂)_{b}R₄,
b is 0, 1 or 2, and
R₄ is C₁-C₄alkoxy, or
when a is 2,
X is C₂-C₈alkylene or -CH₂CH₂(OCH₂CH₂)_{b}-, wherein b is as defined above, or
when a is 3,
X is C₃-C₈alkanetriyl or N(CH₂CH₂-)₃, or
when a is 4,
X is C₄-C₈alkanetetrayl.

7. A process according to claim 6, wherein the protic solvent is methanol, ethanol, isopropanol, n-butanol, ethylene glycol, methyl Cellosolve, cyclohexanol, diethylene glycol or triethanolamine.

8. A process according to claim 7, wherein the protic solvent is ethanol or isopropanol.

9. A process according to any one of claims 1 to 8, wherein the catalyst is a sulfonic acid.

10. A process according to claim 9, wherein the catalyst is p-toluenesulfonic acid, methanesulfonic acid or camphor-10-sulfonic acid.

11. A process according to claim 1, wherein the filtrate is used for a further reaction for the preparation of the compound of formula (1).

12. A process according to any one of claims 1 to 11, wherein the relative molar proportion of the compound of formula (2) to methylamine is from 1:1 to 1:100.

## Revendications

1. Procédé pour la préparation de composés de formule 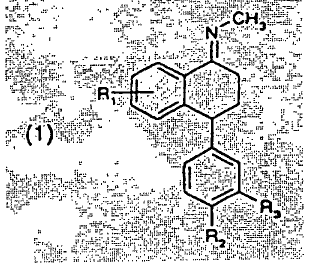 où
R₁, R₂ et R₃, indépendamment les uns des autres, représentent un atome d'hydrogène, un atome d'halogène, un groupe trifluorométhyle ou alkoxy en C₁-C₄,
**caractérisé en ce qu'**on fait réagir
(a) un composé de formule 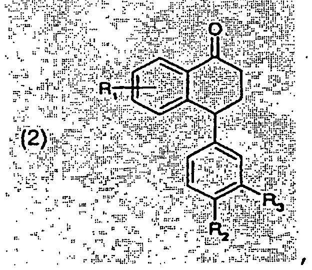 où
R₁, R₂ et R₃ possèdent les significations données à la formule (1),
sur la méthylamine dans un solvant protique, et
(b) on met en oeuvre l'étape réactionnelle (a) en présence d'un catalyseur, le catalyseur étant un acide protonique, un acide de Lewis, un aluminosilicate, une résine échangeuse d'ions, un zéolithe, un phyllosilicate naturel ou un phyllosilicate modifié.

2. Procédé selon la revendication 1, **caractérisé en ce que**
R₁ représente un atome d'hydrogène ou de chlore.

3. Procédé selon l'une des revendications 1 et 2, **caractérisé en ce que** R₂ et R₃, indépendamment l'un de l'autre, représentent un atome d'hydrogène, de chlore ou de brome.

4. Procédé selon l'une des revendications 1 à 3, **caractérisé en ce que**
R₁ représente un atome d'hydrogène et
R₂ et R₃ représentent un atome de chlore.

5. Procédé selon l'une des revendications 1 à 4, **caractérisé en ce que** le solvant protique est un alcool a-valent, a vaut 1, 2, 3 ou 4.

6. Procédé selon la revendication 5, **caractérisé en ce que** le solvant protique est un composé de formule
(3) X(OH)ₐ
où
a vaut 1, 2, 3 ou 4, et
lorsque a vaut 1,
X représente un groupe alkyle en C₁-C₈, cycloalkyle en C₅-C₈ ou -CH₂CH₂(OCH₂CH₂)_{b}R₄,
b vaut 0,1 ou 2, et
R₄ représente un groupe alkoxy en C₁-C₄, ou
lorsque a vaut 2,
X représente un groupe alkylène en C₂-C₈ ou -CH₂CH₂(OCH₂CH₂)_{b-},
ou
lorsque a vaut 3,
X représente un groupe (alcane en C₃-C₈)triyle ou N(CH₂CH₂-)₃, ou
lorsque a vaut 4,
X représente un groupe (alcane en C₄-C₈)tétrayle.

7. Procédé selon la revendication 6, **caractérisé en ce que** le solvant protique est le méthanol, l'éthanol, l'isoproponanol, le n-butanol, l'éthylèneglycol, le méthylcellosolve, le cyclohexanol, le diéthylèneglycol ou la triéthanolamine.

8. Procédé selon la revendication 7, **caractérisé en ce que** le solvant protique est l'éthanol ou l'isoproponanol.

9. Procédé selon l'une des revendications 1 à 8, **caractérisé en ce que** le catalyseur est un acide sulfonique.

10. Procédé selon la revendication 9, **caractérisé en ce que** le catalyseur est l'acide p-toluènesulfonique, l'acide méthanesulfonique ou l'acide camphor-10-sulfonique.

11. Procédé selon la revendication 1, **caractérisé en ce qu'**on utilise le filtrat dans une réaction ultérieure pour la préparation du composé de formule (1).

12. Procédé selon l'une des revendications 1 à 11, **caractérisé en ce que** le rapport quantitatif molaire du composé de formule (2) à la méthylamine est de 1:1 à 1:100.
